# EUROPÄISCHE PATENTSCHRIFT

(11) **EP 1 920 047 B1**
(45) Veröffentlichungstag und Bekanntmachung des Hinweises auf die Patenterteilung: **15.08.2012**
(21) Anmeldenummer: 06792998.4
(22) Anmeldetag: 24.08.2006
(51) Int. Cl.: C12N 5/00, C12Q 1/24, C08F 8/46

(54) **VERFAHREN ZUR SEPARATION VON LEBENDEN ZELLEN**
METHOD FOR THE SEPARATION OF LIVING CELLS
PROCEDE DE SEPARATION DE CELLULES VIVANTES

(30) Priorität: 24.08.2005 DE 102005040259
(43) Veröffentlichungstag der Anmeldung: 14.05.2008
(73) Patentinhaber: Qiagen GmbH, 40724 Hilden (DE)
(72) Erfinder: DENNIG, Jörg, 40764 Langenfeld (DE); MAGYAR, Silvia, 42697 Solingen (DE); ERBACHER, Christoph, 42781 Haan (DE); HIMMELREICH, Ralf, 40764 Langenfeld (DE); PEIST, Ralf, 40229 Düsseldorf (DE)
(86) Internationale Anmeldenummer: PCT/EP2006/065660
(87) Internationale Veröffentlichungsnummer: WO 2007/023181

(56) Entgegenhaltungen:
- WO-A-97/11160
- WO-A-99/07749
- WO-A-03/035888
- WO-A-2005/014801

## Beschreibung

Die vorliegende Erfindung betrifft ein verbessertes Verfahren zur Separation lebender Zellen aus einem Gemisch, das neben lebenden Zellen tote Zellen und gegebenenfalls andere Bestandteile wie Zelltrümmer enthalten kann.

Die Isolierung und Reinigung von Zellpopulationen ist seit langer Zeit in vielen Bereichen der Zellbiologie ein beherrschendes Thema. So kommt es beispielsweise bei der Elektroporation von eukaryontischen Zellen zu deutlichen cytotoxischen Effekten, die viele der anschließenden Analysenmethoden erschweren. Allgemein ergibt sich die Notwendigkeit, nicht nur nach der Elektroporation sondern nach Zellbehandlungen, die unter cytotoxischen Bedingungen durchgeführt werden, die toten Zellen von den lebenden Zellen direkt zu separieren.

Aus dem Stand der Technik sind seit mehreren Jahren Verfahren für derartige Zellseparationen bekannt, die u. a. auf der Trennung durch Dichtezentrifugationstechniken - die auf der Größe und der Dichte beruht - oder z.B. durch Durchflußelektrophoresetechniken - die die Oberflächenladung der Zellen ausnutzt - basieren.

Beispielsweise wird in WO2005/014801 A1 ein Verfahren zur Erhöhung der Zellproduktivität einer Zellkultur durch Entfernung von toten oder sterbenden Zellen aus der Zellkultur beschrieben. Das Verfahren ist dadurch gekennzeichnet, dass die zu entfernenden toten/sterbenden Zellen mit einem Reagenz in Kontakt gebracht werden, das vorzugsweise die toten oder sterbenden Zellen bindet und anschließend diese toten/sterbenden Zellen zusammen mit dem Reagenz aus der Zellkultur entfernt werden. Das Reagenz liegt dabei gebunden oder als Beschichtung auf einem Substrat vor, das aus geeigneten Materialien, wie z.B. Glas, Kunststoff oder Metall besteht. Die Substrate werden dabei zusätzlich mit Antikörper oder anderen Molekülen, die das Reagenz binden können beschichtet.

Neben der Trennung bzw. Isolierung von lebenden Zellen mittels spezifischer Antikörper sind aus dem Stand der Technik Arbeitsweisen bekannt, die beispielsweise die Tendenz apoptotischer bzw. nekrotischer Zellen ausnützen, über die Zellmembran an einen Liganden - wie beispielsweise an Annexin V - zu binden, wobei der Ligand seinerseits beispielsweise an ein magnetisches Partikel als einen festen Träger gekoppelt sein kann.

Zudem sind seit vielen Jahren z.B. zahlreiche Dichtezentrifugationstrennungen bekannt, die mit unterschiedlichen Substanzen zur Ausbildung eines Dichtegradienten arbeiten. Handelsübliche Materialien, die in diesen Verfahren eingesetzt werden können, sind u. a. Ficoll^{®}, Percoll^{®}, Cäsiumchlorid oder Dextran.

Obschon diese Materialien zur Trennung von Zellen durchaus verwendet werden können, weisen sie verschiedene gravierende Nachteile auf, die sie für die erfindungsgemäße Aufgabe als wenig geeignet erscheinen lassen. Diese Nachteile beruhen teilweise auf der Empfindlichkeit der Zellen gegenüber einer hohen osmotischen oder ionischen Belastung sowie gegenüber dem eingesetzten Gradientenmaterial.

So besteht ein schwerwiegender Nachteil dieser Verfahren darin, dass die Zellen für beträchtliche Zeiträume dem Gradientenmaterial ausgesetzt sind, das toxisch sein kann oder wenigstens für die Lebensfähigkeit der Zellen von Nachteil ist. Dies ist insbesondere dann der Fall wenn Gradientenlösungen beispielsweise auf der Basis von Ficoll^{®}, Percoll^{®} oder Dextran zum Einsatz gelangen, die keine physiologischen Lösungen darstellen. Ficoll^{®} ist - wie aus dem Stand der Technik bekannt ist - für die Zellen toxisch und außerdem mutagen. Percoll^{®} verursacht ebenfalls Zellschädigungen. Dextran kann in den zur Gradiententrennung angewendeten Konzentrationen eine Zellschädigung als Ergebnis der auf die Zellen angewendeten osmotischen Belastungen hervorrufen.

Auf der anderen Seite weisen aber auch die alternativ zur Verfügung stehenden und oben genannten Verfahren erhebliche Nachteile auf - so erfordern sie zum Beispiel mehrere Arbeitsschritte und stellen sich als recht aufwendig dar und sind nur schwer automatisierbar.

Die Aufgabe der vorliegenden Erfindung besteht somit darin, die Nachteile der aus dem Stand der Technik bekannten Verfahren wenigstens zum Teil zu vermeiden und ein Verfahren zur Gewinnung von lebenden Zellen zur Verfügung zu stellen, welches insbesondere nicht auf zeit- und arbeitsaufwendige Zentrifugationsschritte zurück greift und welches insbesondere eine Anreicherung oder Isolierung lebender Zellen in möglichst hoher Ausbeute ermöglicht. Eine weitere Aufgabe besteht darin, ein Verfahren zur Verfügung zu stellen, das leicht automatisierbar ist.

Gelöst werden die vorbeschriebenen Aufgaben durch das mit der vorliegenden Erfindung vorgeschlagene Verfahren, bei dem die zu separierenden - lebenden sowie toten - Zellen mit einer Matrix in Kontakt gebracht werden, welche eine Oberfläche aufweist, die lediglich die toten Zellen zu immobilisieren bzw. zu adsorbieren vermag.

Überraschenderweise wurde gefunden, dass tote Zellen an polyanionischen Strukturen aufweisende Polymere, insbesondere an anionische Polymere, die funktionelle Gruppen von Carbonsäuren oder anderen Säuren - wie beispielsweise Sulfon- oder Phosphonsäuren - aufweisen, immobilisiert werden, während lebende Zellen nicht adsorbiert werden.

Das verwendete Zellmaterial weist hierbei vorteilhafterweise Zelltypen wie beispielsweise adhärente Zellen und/oder Suspensionszellen auf. Besonders bevorzugt sind adhärente Primärzellen oder Suspensionszellen wie beispielsweise Immunzellen.

Als Matrices zur Lösung der erfindungsgemäßen Aufgabe sind anionische Strukturen ausbildenden Polymere geeignet, die carboxylierte Polymere, Co- oder Terpolymere auf der Basis von Vinylmethylether, Maleinsäureanhydrid, Styrol, unverzweigten oder verzweigten Alkenen oder Acrylsäure und deren Derivaten aufweisen. Beispielsweise können Copolymere auf der Basis von Styrol, Vinylmethylether, unverzweigten oder verzweigten Alkenen - wie z.B. 1-Octadecen oder Isopropylen und Maleinsäure oder Acrylsäure, wobei die Carboxylfunktionen ggf. in unterschiedlichen Graden verestert sein können - zum Einsatz kommen. Als bevorzugte Polymere seien beispielsweise folgende Acrylsäurealkylester genannt: Methylacrylat, Ethylacrylat, Vinylacrylat, Propylacrylat, Butylacrylat, Hexylacrylat, Octylacrylat, Decylacrylat, Dodecylacrylat, Myristylacrylat, Laurylacrylat, Cetylacrylat, Stearylacrylat, Methylmethacrylat, Ethylmethacrylat, Propylmethacrylat, Butylmethacrylat, Isobutylmethacrylat, Hexylmethacrylat, 2-Ethyl-hexylacrylat, 2-Ethyl-hexylmethacrylat Phenylemethacrylat, Octylmethacrylat, Decylmethacrylat, Dodecylmethacrylat, Myristylmethacrylat, Laurylmethacrylat, Cetylmethacrylat, worunter der Acrylsäuremethylester besonders bevorzugt wird.

Bevorzugt sind die verzweigten oder unverzweigten C₁- bis C₁₂-Alkylester der Acryloder Methacrylsäure.

C₁- bis C₁₂-Alkylester steht im Sinne der vorliegenden Erfindung für einen Alkylester, der in der Alkyl-Partialstruktur des ursprünglichen Alkanol-Teils ein bis zwölf Kohlenstoffatome aufweist.

Allgemein sind im Sinne der vorliegenden Erfindung - sofern nicht anders angegeben - C₁- bis C₆-Alkylgruppen bevorzugt.
C₁-C₆-Alkyl - oder allgemein: Alkyl bzw. Alklyrest - steht im Allgemeinen für einen verzweigten oder unverzweigten Kohlenwasserstoffrest mit 1 bis 6 Kohlenstoffatom(en), der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:
Methyl, Ethyl, Propyl, 1-Methylethyl (iso-Propyl), Butyl, 1-Methylpropyl, 2-Methylpropyl-1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethytbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2methyl-propyl.

Namentlich seien zum Beispiel insbesondere Methylmethacrylat, Ethylmethacrylat, Butylmethacrylat, 2-Ethylhexylmethacrylat, Methylacrylat, Ethylacrylat, Butylacrylat und 2-Ethylhexyacrylat oder Maleinsäurealkylester - wie z.B. insbesondere der Methylester der Maleinsäure - genannt.

Daneben ist es möglich, andere Polylanionenstrukturen ausbildende Polymere einzusetzen, die beispielsweise Sulfon- oder Phosphonsäuregruppen aufweisen; stellvertretend seien als Beispiele Polystyrolsulfonsäuren oder Polystyrolphosphonsäuren in Form ihrer Homopolymere oder als Bestandteile von Copolymeren genannt.

Auch bei diesen Polymeren bzw. Copolymeren kann ggf. ein Teil der Sulfonsäuregruppen oder der Phosphonsäuregruppen verestert sein.

Daneben kann die Phenyl-Partialstruktur aller auf Polystyrol basierenden Polymere ggf. substituiert sein. Dabei bedeutet eine ggf. substituierte Phenylgruppe eine Phenylgruppe, die gegebenenfalls unabhängig voneinander mit einem, zwei oder drei Substituenten, ausgewählt aus Alkyl- (wie oben definiert), Halogenalkylresten, Halogenatomen (Fluor, Chlor, Brom oder Iod), Nitro-, Cyanogruppen, -OR (wobei R ein Wasserstoffatom oder Alkylrest ist), -NRR' (wobei R und R' unabhängig voneinander jeweils ein Wasserstoffatom oder Alkylrest sind), -COOR (wobei R ein Wasserstoffatom oder Alkylrest ist) oder -CONR'R" (wobei R' und R" unabhängig aus Wasserstoffatomen oder Alkylresten ausgewählt sind), substituiert ist. Letztendlich können auch Mischungen aller genannten Polymere bzw. Copolymere eingesetzt werden.

Das zur Ausbildung einer polyanionischen Struktur befähigte Copolymer wird in einer bevorzugten Ausführungsform der vorliegenden Erfindung insbesondere durch ein carboxyliertes Polymer auf der Basis von Styrol und Maleinsäureanhydrid verkörpert. Dabei weist das Copolymer 5 bis 95 Gew.-%, bevorzugt 25 bis 95 Gew.-% und besonders bevorzugt 50 bis 95 Gew.-% an Maleinsäureeinheiten auf.

Das zur Ausbildung einer polyanionischen Struktur befähigte Copolymer wird in einer weiteren bevorzugten Ausführungsform der vorliegenden Erfindung insbesondere durch ein carboxyliertes Polymer auf der Basis von Methylvinylether und Maleinsäure verkörpert.

Bei allen Maleinsäure/Vinylether-Copolymeren kann gegebenenfalls die Vinylalkylether-Partialstruktur als auch die Maleinsäure-Partialstruktur unabhängig voneinander mit einem, zwei, drei oder vier Substituenten - bezogen auf das jeweilige Monomer - ausgewählt aus Alkylresten (wie oben definiert), Halogenalkylresten, Halogenatomen, Nitro-, Cyanogruppen, -OR (wobei R ein Wasserstoffatom oder Alkylrest ist), -NRR' (wobei R und R' unabhängig voneinander jeweils ein Wasserstoffatom oder Alkylrest sind), -COOR (wobei R ein Wasserstoffatom oder Alkylrest ist) oder -CONR'R" (wobei R' und R" unabhängig aus Wasserstoffatomen oder Alkylresten ausgewählt sind), substituiert sein.

Weiterhin können in den Copolymeren - zum Beispiel in -
Poly(methylvinylether-alt-maleinsäure) 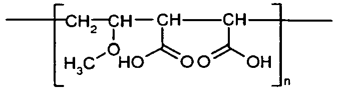 die Carboxylgruppen ggf. ganz oder teilweise - mit einer Alkylgruppe vorzugsweise einer Methylgruppe - verestert sein.

### Daneben finden bevorzugt

Poly(isobutylen-alt-maleinsäure) MW: 3.250.000
Polystyrol-co-maleinsäure, 50 Gewichtsprozent Maleinsäure
Polystyrol-co-maleinsäure, 25 Gewichtsprozent Maleinsäure
Polystyrol-co-maleinsäure, 14 Gewichtsprozent Maleinsäure
Polyisopren-graft-maleinsäure 7 Gewichtsprozent Maleinsäure
Poly(maleinsäure-1-octadec-1-en) im Molverhältnis 1:1
Poly(methylvinylether-alt maleinsäure) MW: 1.250.000
Polystyrol-alt-maleinsäure teilweise als Methylester
Poly(acrylsäure-co-acrylsäuremethylester)
Polystyrolsulfonsäure
Verwendung.

Erfindungsgemäß können Poly(methylvinylether-alt-maleinsäure)-Copolymere mit einem Molekulargewicht in einem Bereich von 1.0·10³ bis 2.5·10⁶, bevorzugt 1.0·10⁴ bis 2.2·10⁶ und besonders bevorzugt in einem Bereich von 1.9·10⁶ bis 2.1·10⁶ eingesetzt werden.

Ganz besonders wird Poly(methyl vinyl ether-alt-maleinsäure) [CAS-Nummer: 25 153-40-6] mit einem Molekulargewicht von MW= 1.980.000 bevorzugt.

Die Herstellung der erfindungsgemäß zum Einsatz kommenden anionischen bzw. carboxylierten Polymere bzw. Copolymere ist aus dem Stand der Technik wohlbekannt. Da diese Polymere bereits in einer Vielzahl anderer technischer Anwendungen eingesetzt werden, sind sie zu einem großen Teil auch kommerziell erhältlich.

Weitere gewünschte Polymere oder Copolymere können mit den aus dem Stand der Technik wohlbekannten Verfahren - z.B. über eine radikalische Polymerisation - mit - ggf. geschützten - Carboxylgruppen-aufweisenden Monomeren hergestellt werden.

Die polyanionischen bzw. carboxylierten Polymere können auf vielfältige Art und Weise auf die Begrenzungswand/-wände eines geeigneten Reaktionsgefäßes aufgebracht werden, z.B. in flüssigem Zustand, oder sie können in Form einer Suspension in dem Gemisch aus lebenden oder toten Zellen zugegen sein oder in Form eines sog. Dippsticks vorliegen.

So kann die Beschichtung auch mit einer Polymerschmelze zu der gewünschten funktionalisierten Schicht führen. Daneben besteht die Möglichkeit - sofern das Reaktionsgefäß mittels Spritzguss hergestellt wird - ein oder mehrere Polymer(e) als Additiv vor dem Spritzguss der für den Spritzguss vorgesehenen Masse zuzufügen. Ebenso besteht die Möglichkeit, alle oder bestimmte Teile der Spritzgussform mit dem/den Polymer(en) vor dem Spritzguss zu beschichten, wobei nach dem Ausbilden des Reaktionsgefäßes die gewünschten Beschichtungen erhalten werden.

Des Weiteren besteht die Möglichkeit, eine Folie aus dem die polyanionischen Strukturen ausbildenden Polymeren an der Innenwand des Reaktionsgefäßes anzubringen und z.B. durch sog. Triefzugverfahren in die gewünschte geometrische Form zu bringen. Bevorzugt wird daneben die Beschichtung mit dem polyanionischen Polymer, das in einem geeigneten Lösungsmittel gelöst ist.

Des Weiteren besteht die Möglichkeit, dass zumindest ein Polymer in die Lösung beispielsweise zunächst als in Form eines sog. Precursors eingebracht wird, aus dem erst im Zuge des Separierungsvorgangs das die gewünschte Struktur aufweisende anionische bzw. Carboxylgruppen-aufweisende Polymer gebildet wird.

Daneben besteht weiterhin die Möglichkeit - bei Auswahl dafür geeigneter Polymere, die dem Fachmann bekannt sind - nach der Herstellung des zunächst nicht funktionalisierten Reaktionsgefäßes, die gewünschte Funktionalisierung der Oberfläche mit aus dem Stand der Technik bekannten "nasschemischen Verfahren" (beispielsweise Schwefelsäure/Wasserstoffperoxid) oder zum Beispiel auf dem Wege der Atmosphärendruck-Plasmabehandlung zu erzielen.

In einer weiteren Ausführungsform kann auf eine Beschichtung oder Derivatisierung der Gefäßoberfläche verzichtet werden, wenn das zur Ausbildung von polyanionischen Strukturen befähigte Polymer dem Gemisch (s.o!) zugefügt wird. Die Bindung der toten Zellen findet dann nicht an einer zusammenhängenden Matrixoberfläche, sondern direkt in der flüssigen Phase statt. Dies führt zu Komplexen bestehend aus den toten Zellen und dem carboxylierten Polymeren die an der - flüssigen bzw. dispersen - Matrix adhärieren können. Das anionische bzw. carboxylierte Polymer selbst kann dann beispielsweise nach einem Fällungsschritt von der flüssigen Phase getrennt werden, wonach die in der flüssigen Phase befindlichen lebenden Zellen isoliert werden können oder das Polymer mit den toten Zellen wird beispielsweise an der Wand des Reaktionsgefäßes adsorbiert.

In allen Ausführungsformen bleiben die toten Zellen an der Matrix bzw. an dem in Lösung befindlichen Polymer immobilisiert zurück, während die lebenden Zellen in der flüssigen Phase verbleiben.

Somit ermöglicht das erfindungsgemäße Verfahren zur Separierung lebender Zellen nicht nur eine deutliche Reduktion der Anzahl der Arbeitsschritte, sondern stellt auch eine schonende Behandlung von Zellen sicher, indem es nicht auf die Anwendung toxischer Substanzen zurückgreift bzw. die Zellen nicht einer hohen osmotischen oder ionischen Belastung aussetzt. Vorteilhafterweise kann durch die deutliche Reduktion der Anzahl an Arbeitsschritten das erfindungsgemäße Verfahren auf ein sog. Einschrittverfahren reduziert werden, bei dem insbesondere auf Abtrennungsschritte, wie beispielsweise Zentrifugations- und/oder Filtrationsschritte verzichtet werden kann. Neben der zeitlichen Komponente kann so zudem auch kostenintensives Verbrauchsmaterial wie z.B. Zentrifugations- und/oder Filtrationssäulen etc. eingespart werden.

Daneben besitzt das erfindungsgemäße Verfahren den Vorteil, dass es aufgrund seiner einfachen Durchführbarkeit leicht automatisierbar ist.

Beispielsweise lässt sich das erfindungsgemäße Verfahren prinzipiell wie folgt durchführen:
Ein geeignetes Reaktionsgefäß - zum Beispiel allgemein eine Zellkulturschale (es können aber auch z.B. Multiwell-Platten, Blöcke oder aber Zellkulturflaschen etc. eingesetzt werden) - wird mit einer der oben beschriebenen Polymerlösungen in einem geeigneten Lösungsmittel - wie zum Beispiel einem organischen Lösungsmittel oder in einem wässrigen Lösungsmittel oder in einem Lösungsmittelgemisch - inkubiert. Dabei kann die Inkubation bei jeder Temperatur, die zwischen dem Erstarrungspunkt der Mischung und deren Siedepunkt liegt, erfolgen.

Als organische Lösungsmittel kommen alle gängigen Lösungsmittel in Frage, wie z.B. halogenierte Kohlenwasserstoffe - wie z.B. Dichlormethan, Chloroform oder Tetrachlorkohlenstoff -, Alkohole - wie z.B. Methanol, Ethanol, Isopropylalkohol, Glycol oder Glycerin -, Ether - wie zum Beispiel Diethylether, Glyme oder Diglyme -, Anisol, THF oder Dioxan -, Ketone - wie z.B. Aceton, Methylethylketon oder Cyclohexanon -, Ester - wie z.B. Essigsäureehtylester -, Säureamide - wie z.B. DMF oder HMPT - oder Sulfoxide - wie z.B. DMSO.

Daneben kommen wässrige Lösungsmittel in Frage - bevorzugt Lösungen von Salzen wie z.B. einer wässrigen Lösung von Alkali-, Pseudoalkali oder Erdalkalielementen, insbesondere aber eine wässrige Ammoniumsulfat-Lösung.

Aus dem Stand der Technik ist eine Vielzahl von weiteren geeigneten Lösungsmitteln bekannt, die in Abhängigkeit von dem jeweils eingesetzten Polymer und ggf. von dem zu beschichtenden Material verwendet werden können, wobei das Polymer bzw. Copolymer in Abhängigkeit von seinem jeweiligen Lösungsverhalten in unterschiedlichsten Konzentrationen eingesetzt werden kann. So kann zum Beispiel bei der Verwendung von Poly(methylvinylether-alt-maleinsäure) als carboxyliertem Polymer vorteilhafterweise eine Lösung von beispielsweise 2 mg/ml des Copolymers in einer 1 M wässrigen Ammoniumsulfat-Lösung zum Einsatz gelangen.

Des Weiteren können selbstverständlich auch Mischungen der oben genannten Lösungsmittel eingesetzt werden.

Die Inkubation kann bei der Verwendung von der oben beschriebenen Poly(methylvinylether-alt-maleinsäure) Lösung beispielsweise bei Raumtemperatur (ca. 10 bis 30 °C, vorzugsweise bei 20 °C) über einen Zeitraum von 5 Minuten erfolgen. In Abhängigkeit von den eingesetzten Polymeren bzw. dem zu beschichtenden Material, kann aber auch eine Temperatur in jedem anderen Bereich. über jedwedes andere Zeitintervall gewählt werden.

Die Lösung wird anschließend entfernt und die so beschichtete Zellkulturschale wird ggf. bei erhöhter Temperatur (30 bis 200 °C vorzugsweise 40 bis 100 °C, besonders bevorzugt bei 40 bis 60 °C) bzw. bei entsprechend reduzierter Temperatur im Vakuum über ein ausreichend langes Zeitintervall - beispielsweise bei 50 °C über einen Zeitraum von ca. 12 Stunden - getrocknet.

Es versteht sich von selbst, dass die auch hier beispielhaft angegebenen Konzentrationsangaben, Temperatur- bzw. Zeitintervalle von den jeweils eingesetzten Polymeren bzw. den Lösungsmitteln und ggf. von der Natur des zu beschichtenden Materials abhängen können und in weiten Grenzen den jeweiligen Bedingungen angepasst bzw. variiert werden können.

Zur Durchführung der Zellseparation wird eine - zum Beispiel aus einer Elektroporation stammende - Zellkultur mit einem Serum-haltigen Kulturmedium gemischt und in die nach dem oben beschriebenen Verfahren beschichtete Zellkulturschale überführt und bei einer geeigneten Temperatur - vorteilhafter Weise bei Raumtemperatur - über einen ausreichend langen Zeitraum inkubiert. Danach wird der Zellüberstand abgenommen.

Es ist auch in Bezug auf diesen Teilschritt einsichtig, dass die angegebenen Temperatur- und Zeitintervalle von dem jeweils zum Einsatz kommenden Beschichtungsmaterial bzw. von den in dem Kulturmedium jeweils vorliegenden Bedingungen - Zellkonzentration, pH-Wert etc. - abhängig sein können.

Die gewonnenen Zellen können anschließend weiter passagiert werden.

Die Analyse der Zellen kann mit jedem geeigneten Bestimmungsverfahren - beispielsweise mittels Durchflußcytometrie und durch Mikroskopieren der zurückgehaltenen bzw. der nicht zurückgehaltenen Zellen - erfolgen.

In der Durchflußcytometrie kann beispielsweise durch Anfärben mit Propidiumiodid der Anteil der lebenden wie auch der toten Zellen bestimmt werden.

Den Abbildungen, die einen integralen Bestandteil dieser Patentanmeldung verkörpern, ist folgendes zu entnehmen:

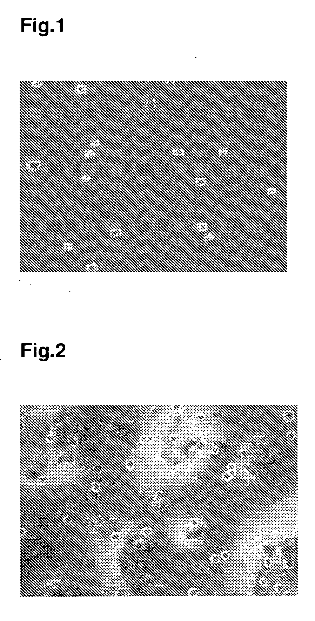

Fig. 1 stellt eine unbeschichtete Zellkulturschale (mikroskopische Aufnahme) im Vergleich zu einer erfindungsgemäß beschichteten Platte Fig. 2 (mikroskopische Aufnahme) dar. Beide Schalen waren mit einer Mischung aus lebenden und toten Zellen inkubiert worden. Anschließend war der Überstand entfernt worden. Aus beiden mikroskopischen Aufnahmen geht deutlich hervor, dass die beschichtete Zellkulturschale - im Gegensatz zur unbeschichteten - verstärkt tote Zellen neben Zelltrümmern adsorbiert hat.

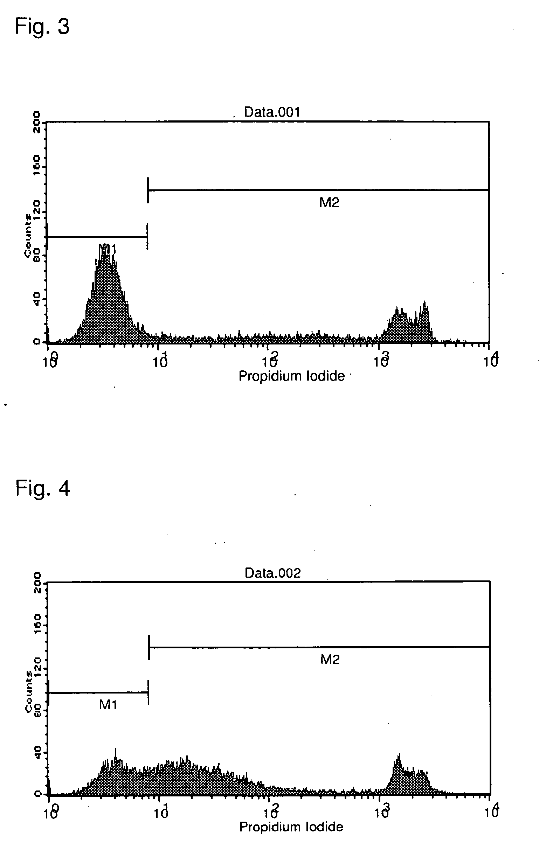

Die Figuren Fig. 3 bis Fig. 5 zeigen drei Histogramme der Durchflußcytometrie (Färbung mit Propidiumiodid).
Dabei zeigt Fig. 3 ein Histogramm der unbehandelten Zellen.
Fig. 4 gibt das Histogramm der Zellen nach der Elektroporation wieder.
Fig. 5 zeigt schließlich das entsprechende Histogramm der erfindungsgemäß aufgereinigten Zellen.

Fig. 6 stellt eine mikroskopische Aufnahme einer Zellkulturschale nach Entfernen des Überstandes dar, die nach dem oben beschriebenen Verfahren behandelt wurde und mit toten Zellen und Zelltrümmern beschichtet ist.

Gegenstand der vorliegenden Erfindung ist somit ein Verfahren zur Separierung lebender von toten Zellen, in dem die Mischung, die lebende sowie tote Zellen enthält, mit einer beschichteten Matrix in Kontakt gebracht wird, welche die toten Zellen adsorbieren kann, und die lebenden Zellen isoliert. Das Matrixmaterial ist dabei ein zur Ausbildung einer polyanionischen Struktur befähigtes carboxyliertes Polymer, Co- oder Terpolymer auf der Basis von Vinylmethylether, Maleinsäureanhydrid, Styrol, unverzweigten oder verzweigten Alkenen oder Acrylsäure und deren Derivaten.

Ein weiterer Aspekt der vorliegenden Erfindung besteht darin, dass das Matrixmaterial im festen Zustand an der Wand des Reaktionsgefäßes oder als Dippstick vorliegt.

Ein weiterer Aspekt der vorliegenden Erfindung besteht darin, dass das Matrixmaterial am Anfang der Zellseparation in einem flüssigen oder dispersen Zustand vorliegt und nach der Separation an den Wänden des Reaktionsgefäßes fixiert bzw. adsorbiert ist oder in ausgefällter oder suspendierter Form vorliegt.

Ein weiterer Aspekt der vorliegenden Erfindung besteht darin, dass das Acrylsäurederivat einen verzweigten oder unverzweigten C₁-C₁₂-Alkylester der Acryloder Methacrylsäure mit einem bis zwölf Kohlenstoffatomen in der Alkanol-Partialstruktur verkörpert.

Ein weiterer Aspekt der vorliegenden Erfindung besteht darin, dass der verzweigte oder unverzweigte Alkylester der Acryl- oder Methacrylsäure Methylmethacrylat, Ethylmethacrylat, Butylmethacrylat, 2- Ethylhexylmethacrylat, Methylacrylat, Ethylacrylat, Butylacrylat und 2- Ethylhexyacrylat ist.

Ein weiterer Aspekt der vorliegenden Erfindung besteht darin, dass das zur Ausbildung einer polyanionischen Struktur befähigte Polymer, Copolymer oder Terpolymer Phosphonsäuregruppen aufweist.

Ein weiterer Aspekt der vorliegenden Erfindung besteht darin, dass das zur Ausbildung einer polyanionischen Struktur befähigte Polymer, Copolymer oder Terpolymer Sulfonsäuregruppen aufweist.

Ein weiterer Aspekt der vorliegenden Erfindung besteht darin, dass das Sulfonsäuregruppen aufweisende Polymer Polystyrolsulfonsäure oder ein Copolymer/Terpolymer mit Polystyrolsulfonsäure ist.

Ein weiterer Aspekt der vorliegenden Erfindung besteht darin, dass das zur Ausbildung einer polyanionischen Struktur befähigte Co- oder Terpolymer ein carboxyliertes Polymer auf der Basis von Styrol und Maleinsäureanhydrid ist.

Ein weiterer Aspekt der vorliegenden Erfindung besteht darin, dass das Copolymere 5 bis 95 Gewichtsprozent Maleinsäureeinheiten enthält.

Ein weiterer Aspekt der vorliegenden Erfindung besteht darin, dass das Copolymere 25 bis 95 Gewichtsprozent Maleinsäureeinheiten enthält.

Ein weiterer Aspekt der vorliegenden Erfindung besteht darin, dass das Copolymere 50 bis 95 Gewichtsprozent Maleinsäureeinheiten enthält.

Ein weiterer Aspekt der vorliegenden Erfindung besteht darin, dass das zur Ausbildung einer polyanionischen Struktur befähigte Co- oder Terpolymer ein carboxyliertes Polymer auf der Basis von Methylvinylether und Maleinsäure ist.

Ein weiterer Aspekt der vorliegenden Erfindung besteht darin, dass das Copolymere Poly(methylvinylether-alt-maleinsäure) ist.

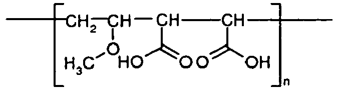

Ein weiterer Aspekt der vorliegenden Erfindung besteht darin, dass das Poly(methylvinylether-alt-maleinsäure)-Copolymere ein Molekulargewicht in einem Bereich von 1.0·10³ bis 2.5·10⁶ aufweist.

Ein weiterer Aspekt der vorliegenden Erfindung besteht darin, dass das Poly(methylvinylether-alt-maleinsäure)-Copolymere ein Molekulargewicht in einem Bereich von 1.0·10⁴ bis 2.2·10⁶ aufweist.

Ein weiterer Aspekt der vorliegenden Erfindung besteht darin, dass das Poly(methylvinylether-alt-maleinsäure)-Copolymere ein Molekulargewicht in einem Bereich von 1.9·10⁶ bis 2.1·10⁶ aufweist.

Ein weiterer Aspekt der vorliegenden Erfindung besteht darin, dass das Poly(methylvinylether-alt-maleinsäure)-Copolymere innerhalb der Fehlergrenzen ein Molekulargewicht von 1.980·10⁶ aufweist.

Ein weiterer Aspekt der vorliegenden Erfindung besteht darin, dass das Copolymer Poly(methylvinylether-alt-maleinsäure) als partieller Alkylester, bevorzugt als C₁-C₆ Alkylester und besonders bevorzugt als Methylester, vorliegt.

Ein weiterer Aspekt der vorliegenden Erfindung besteht darin, dass das Reaktionsgefäß eine Petrischale oder Zellkulturschale, eine Multiwell-Platte eine Zellkuturflasche oder Blöcke verkörpert.

Daneben betrifft die vorliegende Erfindung die Verwendung eines oder mehrerer der vorgenannten Polymere zur Zellseparation, wobei bevorzugt zumindest ein zur Ausbildung einer polyanionischen Struktur befähigtes Polymer, oder Copolymer bzw. Terpolymer zur Separierung von lebenden Zellen verwendet wird, welches besonders bevorzugt ein Polycarboxylat oder ein carboxyliertes Polymer auf der Basis von Vinylmethylether, Maleinsäureanhydrid, Styrol, unverzweigten oder verzweigten Alkenen oder Acrylsäure und deren Derivaten, ist.

Handelt es sich gemäß einer weiteren Ausführung der Erfindung um die Verwendung eines Acrylsäurederivats so verkörpert dieses vorteilhafterweise einen verzweigten oder unverzweigten C₁-C₁₂-Alkylester der Acryl- oder Methacrylsäure mit einem bis zwölf, vorzugsweise einem bis sechs Kohlenstoffatom(en) in der Alkanol-Partialtruktur, wobei der verzweigte oder unverzweigte Alkylester der Acryl- oder Methacrylsäure besonders bevorzugt Methylmethacrylat, Ethylmethacrylat, Butylmethacrylat, 2- Ethylhexylmethacrylat, Methylacrylat, Ethylacrylat, Butylacrylat und 2- Ethylhexyacrylat ist.

Gemäß einem weiteren Aspekt der Erfindung weist das verwendete, zur Ausbildung einer polyanionischen Struktur befähigte. Polymer, Copolymer oder Terpolymer Phosphonsäuregruppen oder Sulfonsäuregruppen auf, wobei das Sulfonsäuregruppen aufweisende Polymer bevorzugt Polystyrolsulfonsäure oder ein Copolymer/Terpolymer mit Polystyrolsulfonsäure ist.

In einem weiteren Aspekt der Erfindung ist das verwendete, zur Ausbildung einer polyanionischen Struktur befähigte Co- oder Terpolymer ein carboxyliertes Polymer auf der Basis von Styrol und Maleinsäureanhydrid, wobei das .Copolymere bevorzugt 5 bis 95 Gewichtsprozent, besonders bevorzugt 25 bis 95 und ganz besonders bevorzugt 50 bis 95 Gewichtsprozent Maleinsäureeinheiten enthält.

Gemäß einem weiteren Aspekt der Erfindung ist das verwendete, zur Ausbildung einer polyanionischen Struktur befähigte Co- oder Terpolymer ein carboxyliertes Polymer auf der Basis von Methylvinylether und Maleinsäure, bevorzugt das Copolymere Poly(methylvinylether-alt-maleinsäure), 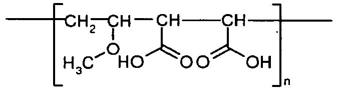 welches vorteilhafterweise ein Molekulargewicht in einem Bereich von 1.0·10³ bis 2.5·10⁶, bevorzugt in einem Bereich von 1.0·10⁴ bis 2.2·10⁶, besonders bevorzugt in einem Bereich von 1.9·10⁶ bis 2.1·10⁶, ganz besonders bevorzugt innerhalb der Fehlergrenzen ein Molekulargewicht von 1.980·10⁶ aufweist.

Gemäß einem weiteren Aspekt der Erfindung liegt das verwendete, Copolymer Poly(methylvinylether-alt-maleinsäure) als partieller Alkylester, bevorzugt als C₁-C₆ Alkylester und besonders bevorzugt als Methylester, vor.

Ein anderer Gegenstand der vorliegenden Erfindung betrifft einen Komplex bestehend aus toten Zellen und einem eine polyanionische Struktur aufweisendem carboxylierten Polymeren, Copolymeren oder Terpolymeren auf der Basis von Vinylmethylether, Maleinsäureanhydrid, Styrol, unverzweigten oder verzweigten Alkenen oder Acrylsäure und deren Derivaten.

Anhand der nachfolgenden Beispiele soll die Erfindung erläutert werden. Diese Beispiele verkörpern lediglich vorteilhafte Ausführungsformen der vorliegenden Erfindung, ohne sie zu beschränken.

### Beispiele

### I Herstellung von verschiedenen Copolymeren

1.) Zu 50 ml Polystyrol-co-maleinsäure mit einem 14%-igen Gewichtsanteil an Maleinsäureanhydrid gelöst in DMSO (20 mg/ml Dimethylsulfoxid) gibt man 250 mg N-(2-Hydroxyethyl-iminodiessigsäure) und erhitzt über einen Zeitraum von 45 min auf 60°C.
   Anschließend verdünnt man mit DMSO 10-fach und erhält somit eine gebrauchsfertige Lösung zur Beschichtung von Reaktionsgefäßen für die Zellseparation. 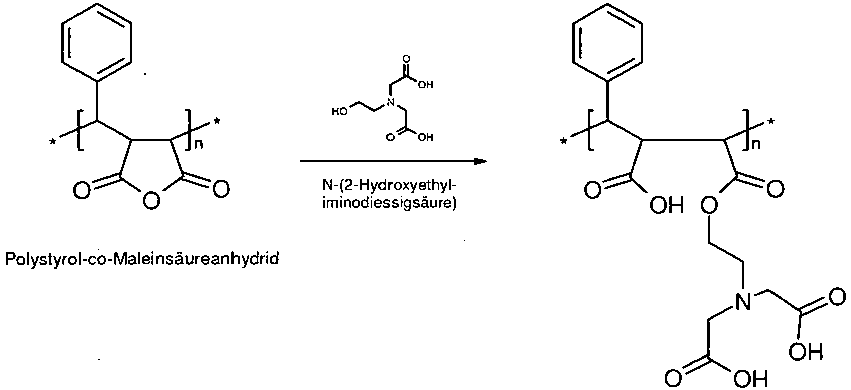
2.) Zu 50ml Polystyrol-co-maleinsäure mit einem 50%-igen Gewichtsanteil an Maleinsäureanhydrid gelöst in DMSO (20mg/ml Dimethylsulfoxid) gibt man 1,3 g N-(2-Hydroxyethyl)-iminobis(methylphosphonsäure) und erhitzt über einen Zeitraum 1 h auf eine Temperatur von 60 °C. Anschließend verdünnt man mit DMSO 10-fach und erhält somit eine gebrauchsfertige Lösung zur Beschichtüng von Reaktionsgefäßen für die Zellseparation. 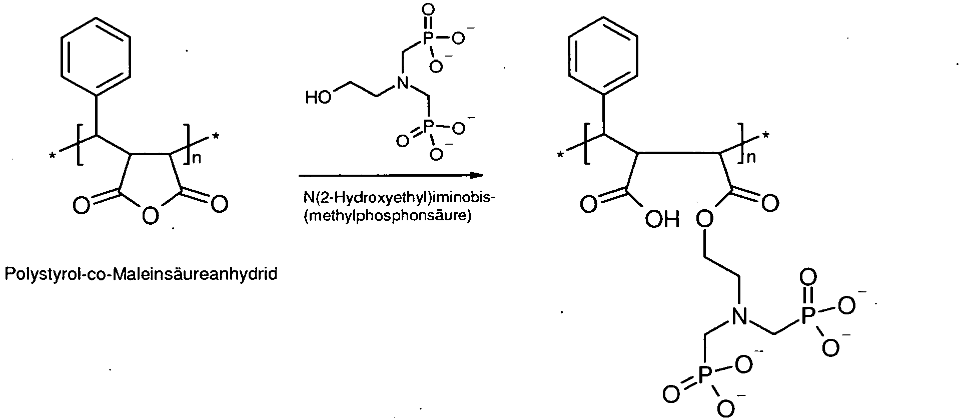
3.) 50 mg (0.877 mmol) N-Me-PVA (N-Methyl-polyvinylamin) wurden in 2 ml Wasser gelöst und nach Zugabe von 1.31 mmol Bromessigsäure (180 mg, 1,5 eq) und 50 µl TEA (Triethylamin) über einen Zeitraum von 12 h geschüttelt. Man erhält so eine gebrauchsfertige Lösung zur Beschichtung von Reaktionsgefäßen für die Zellseparation.
4.) 0.2 g Polyvinylalkohol (MW 9000 -10000) wurden mit 630 mg (0,454 mmol, 0,8 eq.) Bromessigsäure versetzt und nach Zugabe von 1,2 g K₂CO₃ (9,08 mmol, 1,5 eq.) wurde für 12 h geschüttelt. Es resultiert eine gebrauchsfertige Lösung zur Beschichtung von Reaktionsgefäßen für die Zellseparation.

### II Durchführung der Beschichtung

1.) 20 mg Poly(methylvinylether-alt-maleinsäure) wurden in 10 ml VE-Wasser gelöst. Nachdem das Polymer vollständig gelöst war, wurden jeweils 50 µl der wässrigen Polymerlösung in handelsübliche 6-Wellplatten aus Polypropylen pipettiert. Nach 20 min Inkubationszeit bei Raumtemperatur wurde die wässrige Polymerlösung aus den Gefäßen pipettiert. Abschließend wurden die Gefäße einmal mit 120 µl Wasser gespült und bei 40°C getrocknet.
2.) Eine Zellkulturschale (Well) wurde mit 400 µl einer Lösung von Poly(methylvinylether-alt-maleinsäure) (2 mg/ml) in einer 1 M wässrigen Ammoniumsulfat-Lösung bei Raumtemperatur (20 °C) über einen Zeitraum von 5 min inkubiert. Die Lösung wurde anschließend entfernt und die so beschichtete Zellkulturschale bei einer Temperatur 50 °C über einen Zeitraum von 12 h getrocknet.

### III Zellseparation nach Elektroporation

1.) Als Zellmaterial wurden hierbei Suspensionszellen verwendet. Es wurden 200 000 K562-Zellen (humane myeloide Leukämizelllinie) mit 200 µl eines Serum-freien Kulturmediums gewaschen und in 200 µl eines Serum-freien Kulturmediums in einem Elektroporator der Fa. BioRad^{®} gemäß den Herstellervorgaben für die Transfektion von K562-Zellen elektroporiert. Danach erfolgte die Zugabe von 400 µl eines Mediums enthaltend 13,3 % FCS. Die so erhaltene Mischung wird in die nach Beispiel II 2. beschichteten Wells einer 6-Well-Platte überführt, wonach die Zugabe von 1 ml eines Serum-haltigen Mediums erfolgte. Nach der Inkubation ,über einen Zeitraum von 5 min bei Raumtemperatur erfolgte das Abnehmen des Überstandes und dessen Analyse.
Die mikroskopische Analyse ergab, dass eine nach Beispiel II 2. beschichtete Zellkulturschale (Fig. 1, erste mikroskopische Aufnahme) im Vergleich zur unbeschichteten Platte (Fig. 2, zweite mikroskopische Aufnahme) deutlich mehr tote Zellen und Zelltrümmern immobilisiert hat.
Die Ergebnisse der Durchflußcytometrie stellen sich - wie folgt - dar:
Der Prozentsatz lebender Zellen wurde anhand des "Gate M1" in den in den Figs. 3, 4 und 5 dargestellten Histogrammen berechnet. Das Ergebnis ist in der nachfolgenden Tabelle 1 dargestellt:
Tab. 1

| **Art der Zellen** | **% lebende Zellen** |
|---|---|
| Unbehandelte Zellen | 64 |
| Zellen nach der Elektroporation | 36 |
| Aufgereinigte Zellen | 63 |

Den experimentellen Ergebnissen ist zu entnehmen, dass der Anteil an lebenden Zellen nach der Elektroporation von 64 % auf 36 % sinkt, während der Prozentsatz der lebenden Zellen mit 63 % nach der Aufreinigung wieder ungefähr den ursprünglichen Wert erreicht.
Die aufgereinigt Zellpopulation ist daher qualitativ wieder mit den Ursprungszellen vor der Elektroporation vergleichbar.
2.) Auch hierbei wurde als Zellmaterial Suspensionszellen verwendet. Es wurden 200 000 K562-Zellen (humane myeloide Leukämizelllinie) wurden mit 200 µl eines Serum-freien Kulturmediums gewaschen und in 200 µl eines Serum-freien Kulturmediums in einem Elektroporator der Fa. BioRad ® gemäß den Herstellervorgaben für die Transfektion von K562-Zellen elektroporiert. Danach erfolgte die Zugabe von 400 µl eines Mediums enthaltend 13,3 % FCS. Die so erhaltene Mischung wird in die nach Beispiel II 2. beschichteten Wells einer 6-Well-Platte überführt, wonach die Zugabe von 1 ml eines Serum-haltigen Mediums erfolgte. Nach der Inkubation über einen Zeitraum von 5 min bei Raumtemperatur erfolgte das Abnehmen des Überstandes und dessen Analyse.

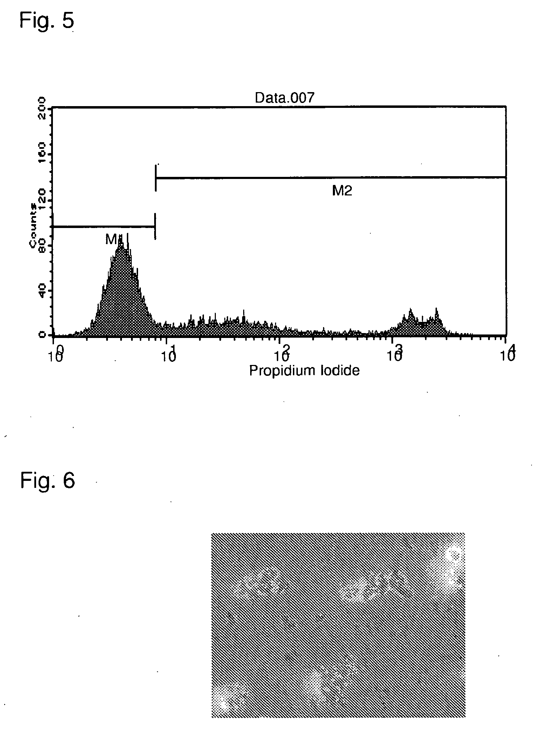

Die in Fig. 6 wiedergegebene (dritte) mikroskopische Aufnahme zeigt, dass die beschichtete Zellkulturschale auch in diesem Experiment tote Zellen neben Zelltrümmern adsorbiert und somit zurückbehält.
Der Prozentsatz lebender Zellen wurde mittels Durchflußcytometrie analog zum vorbeschriebenen ersten Experiment durchgeführt und zeigte das in Tabelle 2 dargestellte Ergebnis:

**Tab. 2**

| **Art der Zellen** | **% lebende Zellen** |
|---|---|
| Unbehandelte Zellen | 77 |
| Zellen nach der Elektroporation | 31 |
| Aufgereinigte Zellen | 56 |

Den experimentellen Ergebnissen ist zu entnehmen, dass der Anteil an lebenden Zellen nach der Elektroporation von 77 % auf 31 % sinkt. Nach der Aufreinigung steigt der Prozentsatz an lebenden Zellen wiederum signifikant auf 56 %. Auch in diesem Experiment ist die Qualität der Zellpopulation nach der Aufreinigung deutlich verbessert.

## Patentansprüche

1. Verfahren zur Separierung lebender von toten Zellen, **dadurch gekennzeichnet, dass** die Mischung, die lebende sowie tote Zellen enthält, mit einer Matrix in Kontakt gebracht wird, welche die toten Zellen adsorbieren kann, und die lebenden Zellen isoliert, **dadurch gekennzeichnet, dass** das Matrixmaterial ein zur Ausbildung einer polyanionischen Struktur befähigtes carboxyliertes Polymer, Co- oder Terpolymer auf der Basis von Vinylmethylether, Maleinsäureanhydrid, Styrol, unverzweigten oder verzweigten Alkenen oder Acrylsäure und deren Derivaten ist.

2. Verfahren nach Anspruch 1 , **dadurch gekennzeichnet, dass** das Matrixmaterial im festen Zustand an einer Wand des Reaktionsgefässes oder als Dippstick vorliegt.

3. Verfahren nach Anspruch 1 oder 2, **dadurch gekennzeichnet, dass** das Matrixmaterial am Anfang der Zellseparation in einem flüssigen oder dispersen Zustand vorliegt und nach der Separation an den Wänden des Reaktionsgefässes fixiert bzw. adsorbiert ist oder in ausgefällter oder suspendierter Form vorliegt.

4. Verfahren nach Anspruch 1 bis 3, **dadurch gekennzeichnet, dass** das Acrylsäurederivat einen verzweigten oder unverzweigten C₁-C₁₂-Alkylester der Acryl- oder Methacrylsäure mit einem bis zwölf, vorzugsweise einem bis sechs Kohlenstoffatom(en) in der Alkanol-Partialstruktur verkörpert.

5. Verfahren nach einem der Ansprüche 1 bis 4, **dadurch gekennzeichnet, dass** das Copolymer Poly(methylvinylether-alt-maleinsäure) ist.

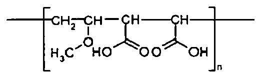

6. Verfahren nach Anspruch 5, **dadurch gekennzeichnet, dass** das Copolymer Poly(methylvinylether-alt-maleinsäure) als partieller Alkylester, bevorzugt als C₁-C₆ Alkylester und besonders bevorzugt als Methylester, vorliegt.

7. Verfahren nach einem der Ansprüche 2 bis 6, **dadurch gekennzeichnet, dass** das Reaktionsgefäss eine Petrischale oder Zellkulturschale, eine Multiwell-Platte, eine Zellkulturflasche oder Blöcke verkörpert.

8. Verwendung eines zur Ausbildung einer polyanionischen Struktur befähigten Polymers, Co- oder Terpolymers zur Separierung von lebenden Zellen, **dadurch gekennzeichnet, dass** die toten Zellen an der Matrix adsorbiert werden, und das zur Ausbildung einer polyanionischen Struktur befähigte Polymer, Co- oder Terpolymer ein carboxyliertes Polymer auf der Basis von Vinylmethylether, Maleinsäureanhydrid, Styrol, unverzweigten oder verzweigten Alkenen oder Acrylsäure und deren Derivaten ist.

9. Verwendung nach Anspruch 8, **dadurch gekennzeichnet, dass** das Acrylsäurederivat einen verzweigten oder unverzweigten C₁-C₁₂-Alkylester der Acryl- oder Methacrylsäure mit einem bis zwölf, vorzugsweise einem bis sechs Kohlenstoffatom(en) in der Alkanol-Partialstruktur verkörpert.

10. Verwendung nach Anspruch 8 oder 9, **dadurch gekennzeichnet, dass** das Copolymer Poly(methylvinylether-alt-maleinsäure) ist.

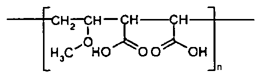

11. Verwendung nach Anspruch 10, **dadurch gekennzeichnet, dass** das Copolymer Poly(methylvinylether-alt-maleinsäure) als partieller Alkylester, bevorzugt als C₁-C₆ Alkylester und besonders bevorzugt als Methylester, vorliegt.

12. Komplex bestehend aus toten Zellen und einem eine polyanionische Struktur aufweisendem Polymeren, Copolymeren oder Terpolymeren, **dadurch gekennzeichnet, dass** das Polymer, Co- oder Terpolymer ein carboxyliertes Polymer auf der Basis von Vinylmethylether, Maleinsäureanhydrid, Styrol, unverzweigten oder verzweigten Alkenen oder Acrylsäure und deren Derivaten ist.

13. Komplex nach Anspruch 12, **dadurch gekennzeichnet, dass** das Acrylsäurederivat einen verzweigten oder unverzweigten C₁-C₁₂-Alkylester der Acryl- oder Methacrylsäure mit einem bis zwölf, vorzugsweise einem bis sechs Kohlenstoffatom(en) in der Alkanol-Partialstruktur verkörpert.

14. Komplex nach Anspruch 12 oder 13, **dadurch gekennzeichnet, dass** das Copolymer Poly(methylvinylether-alt-maleinsäure) ist.

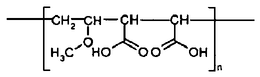

15. Komplex nach Anspruch 14, **dadurch gekennzeichnet, dass** das Copolymer Poly(methylvinylether-alt-maleinsäure) als partieller Alkylester, bevorzugt als C₁-C₆ Alkylester und besonders bevorzugt als Methylester, vorliegt.

## Claims

1. Method for separating living cells from dead cells, **characterized in that** the mixture that contains living and dead cells is brought into contact with a matrix that can adsorb the dead cells and isolates the living cells, **characterized in that** the matrix material is a carboxylated polymer, co- or terpolymer that is capable of forming a polyanionic structure and is based on vinyl methyl ether, maleic anhydride, styrene, unbranched or branched alkenes, or acrylic acid and derivatives thereof.

2. Method according to Claim 1, **characterized in that** the matrix material is present in the solid state on a wall of the reaction vessel or as a dipstick.

3. Method according to Claim 1 or 2, **characterized in that** the matrix material is present at the start of cell separation in a liquid or disperse state and after separation is fixed or adsorbed to the walls of the reaction vessel or is present in precipitated or suspended form.

4. Method according to Claim 1 to 3, **characterized in that** the acrylic acid derivative embodies a branched or unbranched C₁-C₁₂ alkyl ester of acrylic or methacrylic acid having one to twelve, preferably one to six, carbon atoms in the alkanol partial structure.

5. Method according to any one of Claims 1 to 4, **characterized in that** the copolymer is poly(methyl vinyl ether-alt-maleic acid).

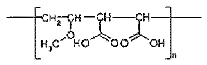

6. Method according to Claim 5, **characterized in that** the copolymer poly(methyl vinyl ether-alt-maleic acid) is present as partial alkyl ester, preferably as C₁-C₆ alkyl ester, and particularly preferably as methyl ester.

7. Method according to any one of Claims 2 to 6, **characterized in that** the reaction vessel embodies a Petri dish or cell culture dish, a multiwell plate, a cell culture bottle or blocks.

8. Use of a polymer, co- or terpolymer capable of forming a polyanionic structure for the separation of living cells, **characterized in that** the dead cells are adsorbed to the matrix and the polymer, co- or terpolymer capable of forming a polyanionic structure is a carboxylated polymer based on vinyl methyl ether, maleic anhydride, styrene, unbranched or branched alkenes, or acrylic acid and derivatives thereof.

9. Use according to Claim 8, **characterized in that** the acrylic acid derivative embodies a branched or unbranched C₁-C₁₂ alkyl ester of acrylic or methacrylic acid having one to twelve, preferably one to six, carbon atoms in the alkanol partial structure.

10. Use according to Claim 8 or 9, **characterized in that** the copolymer is poly(methyl vinyl ether-alt-maleic acid).

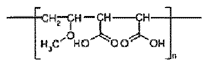

11. Use according to Claim 10, **characterized in that** the copolymer poly(methyl vinyl ether-alt-maleic acid) is present as partial alkyl ester, preferably as C₁-C₆ alkyl ester, and particularly preferably as methyl ester.

12. Complex consisting of dead cells and a polymer, copolymer or terpolymer having a polyanionic structure, **characterized in that** the polymer, co-or terpolymer is a carboxylated polymer based on vinyl methyl ether, maleic anhydride, styrene, unbranched or branched alkenes, or acrylic acid and derivatives thereof.

13. Complex according to Claim 12, **characterized in that** the acrylic acid derivative embodies a branched or unbranched C₁-C₁₂ alkyl ester of acrylic or methacrylic acid having one to twelve, preferably one to six, carbon atoms in the alkanol partial structure.

14. Complex according to Claim 12 or 13, **characterized in that** the copolymer is poly(methyl vinyl ether-alt-maleic acid).

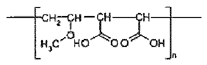

15. Complex according to Claim 14, **characterized in that** the copolymer poly(methyl vinyl ether-alt-maleic acid) is present as partial alkyl ester, preferably as C₁-C₆ alkyl ester, and particularly preferably as methyl ester.

## Revendications

1. Procédé de séparation de cellules vivantes de cellules mortes, **caractérisé en ce que** le mélange, qui contient des cellules vivantes et des cellules mortes, est mis en contact avec une matrice qui peut adsorber les cellules mortes, et isole les cellules vivantes, **caractérisé en ce que** le matériau de matrice est un polymère, co- ou terpolymère carboxylé apte à former une structure polyanionique, à base d'éther de vinyle et de méthyle, d'anhydride de l'acide maléique, de styrène, d'alcènes non ramifiés ou ramifiés ou d'acide acrylique et ses dérivés.

2. Procédé selon la revendication 1, **caractérisé en ce que** le matériau de matrice se présente à l'état solide sur une paroi du récipient de réaction ou sous la forme d'une bandelette.

3. Procédé selon la revendication 1 ou 2, **caractérisé en ce que** le matériau de matrice se présente au début de la séparation des cellules à un état liquide ou dispersé et, après la séparation, est fixé ou adsorbé sur les parois du récipient de réaction ou se présente sous forme précipitée ou suspendue.

4. Procédé selon les revendications 1 à 3, **caractérisé en ce que** le dérivé d'acide acrylique est un ester d'alkyle en C₁-C₁₂ ramifié ou non ramifié de l'acide acrylique ou méthacrylique contenant un à douze, de préférence un à six, atomes de carbone dans la structure partielle alcanol.

5. Procédé selon l'une quelconque des revendications 1 à 4, **caractérisé en ce que** le copolymère est le poly(éther de méthyle et de vinyle-alt-acide maléique)

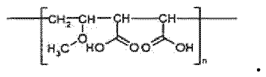

6. Procédé selon la revendication 5, **caractérisé en ce que** le copolymère poly(éther de méthyle et de vinyle-alt-acide maléique) se présente sous la forme d'un ester d'alkyle partiel, de préférence sous la forme d'un ester d'alkyle en C₁-C₆ et de manière particulièrement préférée sous la forme d'un ester de méthyle.

7. Procédé selon l'une quelconque des revendications 2 à 6, **caractérisé en ce que** le récipient de réaction est une boîte de Pétri ou une plaque de culture cellulaire, une plaque multi-puits, une bouteille de culture cellulaire ou des blocs.

8. Utilisation d'un polymère, co- ou terpolymère apte à former une structure polyanionique pour la séparation de cellules vivantes, **caractérisée en ce que** les cellules mortes sont adsorbées sur la matrice, et le polymère, co- ou terpolymère apte à former une structure polyanionique est un polymère carboxylé à base d'éther de vinyle et de méthyle, d'anhydride de l'acide maléique, de styrène, d'alcènes non ramifiés ou ramifiés ou d'acide acrylique et ses dérivés.

9. Utilisation selon la revendication 8, **caractérisée en ce que** le dérivé d'acide acrylique est un ester d'alkyle en C₁-C₁₂ ramifié ou non ramifié de l'acide acrylique ou méthacrylique contenant un à douze, de préférence un à six, atomes de carbone dans la structure partielle alcanol.

10. Utilisation selon la revendication 8 ou 9, **caractérisée en ce que** le copolymère est le poly(éther de méthyle et de vinyle-alt-acide maléique)

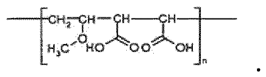

11. Utilisation selon la revendication 10, **caractérisée en ce que** le copolymère poly(éther de méthyle et de vinyle-alt-acide maléique) se présente sous la forme d'un ester d'alkyle partiel, de préférence sous la forme d'un ester d'alkyle en C₁-C₆ et de manière particulièrement préférée sous la forme d'un ester de méthyle.

12. Complexe constitué de cellules mortes et d'un polymère, copolymère ou terpolymère présentant une structure polyanionique, **caractérisé en ce que** le polymère, co- ou terpolymère est un polymère carboxylé à base d'éther de vinyle et de méthyle, d'anhydride de l'acide maléique, de styrène, d'alcènes non ramifiés ou ramifiés ou d'acide acrylique et ses dérivés.

13. Complexe selon la revendication 12, **caractérisé en ce que** le dérivé d'acide acrylique est un ester d'alkyle en C₁-C₁₂ ramifié ou non ramifié de l'acide acrylique ou méthacrylique contenant un à douze, de préférence un à six, atomes de carbone dans la structure partielle alcanol.

14. Complexe selon la revendication 12 ou 13, **caractérisé en ce que** le copolymère est le poly(éther de méthyle et de vinyle-alt-acide maléique)

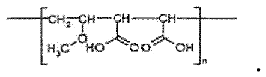

15. Complexe selon la revendication 14, **caractérisé en ce que** le copolymère poly(éther de méthyle et de vinyle-alt-acide maléique) se présente sous la forme d'un ester d'alkyle partiel, de préférence sous la forme d'un ester d'alkyle en C₁-C₆ et de manière particulièrement préférée sous la forme d'un ester de méthyle.
